# EUROPÄISCHE PATENTSCHRIFT

(11) **EP 2 422 693 B1**
(45) Veröffentlichungstag und Bekanntmachung des Hinweises auf die Patenterteilung: **28.11.2018**
(21) Anmeldenummer: 10174253.4
(22) Anmeldetag: 27.08.2010
(51) Int. Cl.: A61B 5/145, A61B 5/00

(54) **Vorrichtung und Verfahren zur Durchführung mindestens einer medizinischen Funktion**
Device and method for performing at least one medical function
Dispositif et procédé d'exécution d'au moins une fonction médicale

(43) Veröffentlichungstag der Anmeldung: 29.02.2012
(73) Patentinhaber: Roche Diabetes Care GmbH, 68305 Mannheim (DE); F. Hoffmann-La Roche AG, 4070 Basel (CH)
(72) Erfinder: Frey, Stephan-Michael, 64347 Griesheim (DE); Walter, Helmut, 64646 Heppenheim (DE); Braun, Jürgen, 71139 Ehningen (DE); Pfrang, Jürgen, 93183 Kallmünz (DE)
(74) Vertreter: Herzog, Fiesser & Partner Patentanwälte PartG mbB

(56) Entgegenhaltungen:
- EP-A1- 1 988 394
- DE-A1- 10 341 093
- US-A1- 2004 204 673
- US-A1- 2005 159 752
- US-A1- 2008 194 961
- US-A1- 2008 284 372
- US-A1- 2010 191 078

## Beschreibung

### Gebiet der Erfindung

Die Erfindung betrifft eine Vorrichtung zur Durchführung mindestens einer medizinischen Funktion an einem Benutzer. Weiterhin betrifft die Erfindung ein Verfahren zur Entsorgung von Komponenten eines Ansteuer- und Auswerteteils einer erfindungsgemäßen Vorrichtung, insbesondere eines Einwegteils des Ansteuer- und Auswerteteils. Erfindungsgemäße Vorrichtungen zur Durchführung mindestens einer medizinischen Funktion werden insbesondere im Bereich der medizinischen Diagnostik oder Therapeutik eingesetzt. So kann die Vorrichtung beispielsweise als Sensorvorrichtung ausgestaltet sein, um mindestens eine Körperfunktion des Benutzers zu erfassen. Beispielsweise kann die Vorrichtung als Sensorvorrichtung zum qualitativen und/oder quantitativen Nachweis mindestens eines Analyten in einer Körperflüssigkeit des Benutzers ausgestaltet sein. Alternativ oder zusätzlich kann die Vorrichtung jedoch auch beispielsweise als Medikationsvorrichtung zur Verabreichung mindestens einer Medikation an den Benutzer ausgestaltet sein. Ein besonderer Schwerpunkt der Anwendung der vorliegenden Erfindung liegt auf einer LangzeitÜberwachung kontinuierlicher oder diskontinuierlicher Art mindestens einer Analytkonzentration in mindestens einer Körperflüssigkeit wie beispielsweise interstitieller Flüssigkeit oder Blut. Als zu überwachende Analyte kommen beispielsweise Glucose, Cholesterin, Lactat, allgemein Metabolite oder andere Arten von Analyten oder Analytkombinationen in Betracht. Prinzipiell ist die vorliegende Erfindung auch auf andere medizinische Bereiche, beispielsweise in der Diagnostik, der Therapeutik oder der Chirurgie, anwendbar.

### Stand der Technik

Aus dem Stand der Technik sind zahlreiche medizinische Vorrichtungen mit diagnostischen, therapeutischen oder chirurgischen Funktionen bekannt. Insbesondere die Überwachung und/oder Beeinflussung bestimmter Körperfunktionen, insbesondere die Überwachung einer oder mehrerer Konzentrationen bestimmter Analyte, spielt bei der Vorbeugung und Behandlung verschiedener Krankheiten eine wesentliche Rolle. Ohne Beschränkung weiterer möglicher Anwendungen wird die Erfindung im Folgenden im Wesentlichen unter Bezugnahme auf eine Blutglucoseüberwachung beschrieben, insbesondere unter Bezugnahme auf eine kontinuierliche Langzeit-Blutglucoseüberwachung über mehrere Stunden, Tage, Wochen oder sogar Monate. Grundsätzlich ist die Erfindung jedoch auch auf andere Arten von Analytüberwachungen und/oder die Überwachung anderer Arten von Körperfunktionen sowie auf andere Gebiete der Medizin übertragbar. Insbesondere kann die Erfindung auch auf die medizinische Therapeutik übertragen werden, beispielsweise auf Medikationspumpen wie beispielsweise Insulinpumpen.

Neben so genannten Punktmessungen eines oder mehrerer Analyte, bei welchen einem Benutzer gezielt eine Probe einer Körperflüssigkeit entnommen wird, etablieren sich zunehmend auch kontinuierliche Messungen. So etabliert sich beispielsweise eine kontinuierliche Glucosemessung im Interstitium (auch als Continuous Monitoring, CM, bezeichnet) in jüngerer Vergangenheit als wichtige Methode zum Management, zur Überwachung und zur Steuerung beispielsweise eines Diabetes-Status. Vorerst beschränkt sich diese kontinuierliche Überwachung in der Regel auf Diabetiker vom Typ I, also Diabetiker, welche üblicherweise auch eine Insulinpumpe tragen. Mittlerweile kommen dabei in der Regel direkt implantierte elektrochemische Sensoren zum Einsatz, welche häufig auch als nadelartige Sensoren (needle type sensors, NTS) bezeichnet werden. Dabei wird der aktive Sensorbereich direkt an den Messort gebracht, welcher in der Regel im interstitiellen Gewebe angeordnet ist, und beispielsweise unter Verwendung eines Enzyms (beispielsweise Glucoseoxidase, GOD) Glucose in elektrische Ladungen umsetzt, die im Verhältnis zur Glucose-Konzentration stehen und als Messgröße verwendet werden können. Beispiele derartiger transkutaner Messsysteme sind in US 6,360,888 B1 oder in US 2008/0242962 A1 beschrieben.

Aktuelle Continuous Monitoring-Systeme sind somit zumeist transkutane Systeme. Dies bedeutet in der Regel, dass der eigentliche Sensor unterhalb der Haut des Benutzers angeordnet ist. Ein Auswerte- und Steuerteil des Systems, welches auch als Patch bezeichnet wird, befindet sich jedoch in der Regel außerhalb des Körpers des Benutzers, also außerhalb des menschlichen oder tierischen Körpers. Der Sensor wird dabei in der Regel mittels eines Insertionsbestecks appliziert und in das Körpergewebe insertiert, welches exemplarisch ebenfalls in US 6,360,888 B1 beschrieben wird. Auch andere Arten von Insertionsbestecken sind bekannt. Die Tragedauer eines Sensors beträgt in der Regel ca. 1 Woche, wobei jedoch auch längere Tragedauern, beispielsweise bis hin zu einem oder mehreren Monaten, möglich sind. Danach lassen in der Regel Einflüsse, wie beispielsweise ein Enzymverbrauch und/oder eine Abkapselung im Körper, die Sensitivität des Sensors abfallen, und damit ist ein Ausfall des Sensors zu erwarten. Die Verlängerung der Tragedauer stellt ein aktuelles Entwicklungsgebiet dar. Dies bedeutet jedoch, dass der Sensor und optional mit ihm unmittelbar in Verbindung stehende Komponenten, wie beispielsweise eine Insertionsnadel, als austauschbare Bauelemente ausgestaltet werden sollten. Dementsprechend stellen der Sensor und in der Regel weitere austauschbare Komponenten der Vorrichtung einen so genannten Einwegteil (Disposable) dar. Das Auswerte- und Steuerteil des Systems oder wichtige, teure Komponenten desselben (wie beispielsweise hochohmige Verstärker-Eingangsstufen und ähnliche aktive Bauelemente) werden jedoch in der Regel wiederverwendet, so dass die Vorrichtung in vielen Fällen mindestens einen Mehrwegteil (Reusable) umfasst.

Bei implantierbaren Sensoren umfasst der Einwegteil in der Regel ein so genanntes Bodymount, welches auf einer Hautoberfläche des Benutzers fixiert werden kann, beispielsweise mittels eines Pflasters, und mit welchem der in das Körpergewebe insertierte Sensor verbunden werden kann oder verbunden ist. Mit diesem Bodymount wird dann das Reusable verbunden, welches die wesentlichen Teile der Ansteuer- und/oder Auswerteelektronik für die Messung der Analytkonzentration enthält. Im Bodymount selbst können jedoch Komponenten angeordnet werden. So kann beispielsweise mindestens eine Batterie in dem Bodymount angeordnet werden, so dass mit dem Austausch eines Bodymounts und dem Zusammenfügen eines neuen Bodymounts mit dem Reusable die Vorrichtung gleichzeitig auch mit einer neuen Energiequelle ausgestattet wird.

Diese Trennung von Disposable und Reusable bewirkt, dass der Benutzer nicht gezwungen ist, während der Benutzung des Sensors die Batterien entweder auszutauschen oder aufzuladen, da mit dem Austausch des Bodymounts mit dem integrierten Batteriefach ohnehin ein Austausch der Batterien erfolgen muss. Allerdings müssen gemäß jüngeren Umweltrichtlinien bei mit Batterien betriebenen elektrischen Geräten die Batterien vom Kunden separat entsorgt werden können. Somit besteht eine Herausforderung darin, eine Vorrichtung zu schaffen, welche insbesondere für einige Tage am Körper des Benutzers getragen werden kann und somit gegenüber allen wirkenden Umwelteinflüssen (Feuchte, Temperaturschwankungen, mechanischer Einfluss) abgeschirmt ist, wobei jedoch gleichzeitig gewährleistet werden soll, dass der Benutzer nach der Tragezeit die Batterien selbst entsorgen kann.
Aus dem Stand der Technik sind grundsätzlich verschiedene Gehäuse für medizinische Vorrichtungen bekannt, die entweder vollständig eingeschweißte Batterien aufweisen oder Batteriefächer, die mehrmals geöffnet und geschlossen werden können. Beispielsweise beschreibt US 2009/0253960 A1 eine Antenneneinheit mit einer Antenne zum Empfang von in-vivo-Informationen eines Benutzers. Dabei wird eine eingegossene Antenne verwendet. Unter anderem werden dabei flexible Batterien vorgeschlagen, wobei die Vorteile der flexiblen Deformation der eingegossenen Batterie auf dem Körper des Benutzers hervorgehoben werden. Eine separate Entsorgung der Batterie nach der Lebensdauer des medizinischen Systems ist jedoch nicht oder nur schwer möglich.
Weiterhin sind aus dem Stand der Technik Systeme bekannt, bei welchen eine Batterie auswechselbar gestaltet ist. Beispielsweise werden in US 6,498,951, in US 6,749,587 und in US 6,175,752 B1 verschiedene Systeme dargestellt, bei welchen Batterien auswechselbar gestaltet sind. Zudem, wird in US 6,749,587 ein Batteriefach mit einem Deckel verwendet, oder alternativ, wird die Batterieaufnahme samt Batterie zusammen mit einem Einwegteilgehäuse entsorgt. In US2010191078, US 6,498,951 und US 6,175,752 werden Gehäuse mit zwei Komponenten beschrieben, welche lösbar miteinander verbunden werden können und bei denen eine hermetische Versiegelung des Batteriefachs vorgesehen ist.

Diese bekannten Ansätze weisen jedoch Nachteile auf, da die oben beschriebenen technischen Herausforderungen nicht vollständig gelöst werden. So ist im einen Fall die Batterie nicht separat entsorgbar, und im anderen Fall stellen die auswechselbare Ausgestaltung der Batterie und die damit verbundenen technischen Anforderungen an das Batteriefach erhebliche technische Herausforderungen dar. Insbesondere muss bei reversibel zu öffnenden Batteriefächern nach wie vor eine Feuchtigkeitsdichtigkeit gewährleistet sein, da insbesondere bei am Körper eines Benutzers tragbaren medizinischen Vorrichtungen diesbezüglich hohe Anforderungen zu stellen sind.

### Aufgabe der Erfindung

Es ist dementsprechend eine Aufgabe der vorliegenden Erfindung, eine Vorrichtung zur Durchführung mindestens einer medizinischen Funktion an einem Benutzer bereitzustellen, welche die Nachteile bekannter Vorrichtungen der genannten Art vermeidet. Insbesondere soll ein Benutzer einerseits nicht gezwungen sein, während der Benutzung Batterien auszutauschen oder aufzuladen, und andererseits soll nach der Benutzung eine separate Entsorgung der Batterien möglich sein.

### Offenbarung der Erfindung

Diese Aufgabe wird gelöst durch eine Vorrichtung und ein Verfahren mit den Merkmalen der unabhängigen Patentansprüche. Vorteilhafte Weiterbildungen der Erfindung, welche einzeln oder in beliebiger Kombination realisierbar sind, sind in den abhängigen Ansprüchen dargestellt.

Es wird eine Vorrichtung zur Durchführung mindestens einer medizinischen Funktion an einem Benutzer vorgeschlagen. Unter einer medizinischen Funktion ist dabei eine diagnostische, eine therapeutische oder eine chirurgische Funktion zu verstehen. Auch eine Kombination verschiedener Funktionen ist möglich. Die Vorrichtung kann insbesondere als kompaktes Gerät ausgestaltet sein, welches von dem Benutzer mitgeführt werden kann. Insbesondere kann die Vorrichtung ausgestaltet sein als kompaktes Gerät, welches ganz oder teilweise an oder in einem Körper des Benutzers getragen werden kann.

Die Vorrichtung weist mindestens ein medizinisches Funktionselement auf, welches eingerichtet ist, um die mindestens eine medizinische Funktion durchzuführen. Das medizinische Funktionselement ist dementsprechend auf die mindestens eine medizinische Funktion angepasst. Beispielsweise kann die medizinische Funktion, wie oben ausgeführt, mindestens eine diagnostische Funktion umfassen, so dass das medizinische Funktionselement beispielsweise ganz oder teilweise als Diagnoseelement ausgestaltet sein kann, beispielsweise indem dieses mindestens einen Sensor zur Erfassung mindestens einer Körperfunktion umfasst. Dabei kann es sich grundsätzlich um beliebige Körperfunktionen handeln. Insbesondere kann es sich bei dem Sensor um mindestens einen Sensor zum quantitativen und/oder qualitativen Erfassen mindestens eines Analyten in mindestens einer Körperflüssigkeit handeln, beispielsweise einen oder mehrere der oben beschriebenen Sensoren, insbesondere einen Sensor zur Erfassung einer Blutglucosekonzentration in einer Körperflüssigkeit. Umfasst die medizinische Funktion beispielsweise eine therapeutische Funktion, so kann das medizinische Funktionselement beispielsweise mindestens einen therapeutischen Aktor umfassen, welcher eingerichtet sein kann, um auf mindestens eine Körperfunktion des Benutzers einzuwirken und/oder auf den Körper des Benutzers einen Reiz auszuüben. Beispielsweise kann dieser Aktor mindestens eine Medikationsvorrichtung umfassen, beispielsweise mindestens eine Insulinpumpe. Alternativ oder zusätzlich zu den genannten medizinischen Funktionen und/oder den genannten medizinischen Funktionselementen kann die Vorrichtung jedoch auch andere und/oder weitere medizinische Funktionen und/oder medizinische Funktionselemente umfassen.

Die Erfindung wird im Folgenden im Wesentlichen beschrieben unter Bezugnahme auf ein bevorzugtes Ausführungsbeispiel, bei welchem die Vorrichtung eine Vorrichtung zur qualitativen und/oder quantitativen Erfassung eines Analyten in einer Körperflüssigkeit ist, mit einem auf einer Hautoberfläche des Benutzers aufbringbaren Gehäuse und einem in ein Körpergewebe des Benutzers insertierbaren Sensor. Als Beispiel für derartige Vorrichtungen, mit Ausnahme der erfindungsgemäßen Ausgestaltung, kann auf die obige Beschreibung bekannter Sensoren, insbesondere bekannter Langzeitsensoren, verwiesen werden. Allgemein kann die medizinische Funktion jedoch ausgewählt sein aus einer diagnostischen, einer therapeutischen und einer chirurgischen Funktion oder Kombinationen der genannten Funktionen.

Die Vorrichtung weist mindestens ein Auswerte- und Steuerteil auf. Dieses Auswerte- und Steuerteil kann insbesondere ganz oder teilweise als so genannter Patch ausgestaltet sein, welcher direkt oder indirekt, beispielsweise unter Zwischenschaltung mindestens eines Pflasters, auf einer Hautoberfläche des Benutzers getragen werden. Unter einem Auswerte- und Steuerteil ist dabei im Rahmen der vorliegenden Erfindung ein einteilig oder auch mehrteilig ausgestaltetes Bauelement zu verstehen, welches eingerichtet ist, um das medizinische Funktionselement mit mindestens einem Signal, beispielsweise einem elektrischen Signal, und/oder mindestens einer Energie, beispielsweise einem Strom und/oder einer Spannung, zu beaufschlagen und/oder das medizinische Funktionselement in anderer Weise zur Durchführung seiner medizinischen Funktion anzuregen, und/oder welches eingerichtet ist, um mindestens ein elektrisches Signal des medizinischen Funktionselements aufzunehmen, beispielsweise ein Spannungssignal und/oder ein Stromsignal. Aufgenommene Signale können beispielsweise einfach erfasst und/oder ganz oder teilweise umgewandelt werden und/oder gespeichert werden und/oder auch bereits zumindest teilweise ausgewertet oder verarbeitet werden. Im Falle eines Sensorelements als medizinisches Funktionselement kann das Auswerte- und Ansteuerteil beispielsweise eingerichtet sein, um das Sensorelement mit einer Spannung und/oder einem Strom zu beaufschlagen und/oder kann beispielsweise einen Messwertverstärker und/oder einen Potentiostaten umfassen, um Spannungs- oder Stromsignale aufzunehmen. Im Falle einer Medikationsvorrichtung als medizinisches Funktionselement kann das Auswerte- und Ansteuerteil beispielsweise auch eine oder mehrere fluidische, mechanische oder fluid-mechanische Komponenten umfassen, beispielsweise eine Pumpe, ein Ventil, ein Rohrleitungssystem oder Kombinationen der genannten und/oder anderer Elemente. Verschiedene Ausgestaltungen sind möglich.

Das Auswerte- und Ansteuerteil kann einstückig oder auch mehrstückig ausgestaltet sein. Beide Möglichkeiten sollen erfindungsgemäß möglich sein. Ohne Beschränkung anderer Ausgestaltungen wird die Erfindung im Folgenden im Wesentlichen unter Bezugnahme auf mehrteilige Ausgestaltungen des Auswerte- und Ansteuerteils beschrieben. So kann das Auswerte- und Ansteuerteil insbesondere mindestens einen Einwegteil und mindestens einen Mehrwegteil aufweisen. Unter einem Einwegteil wird dabei ein Teil der Vorrichtung, insbesondere des Auswerte- und Ansteuerteils, verstanden, welcher bei einer bestimmungsgemäßen Verwendung der Vorrichtung lediglich einmal oder wenige Male verwendet wird, wohingegen der Mehrwegteil bei der bestimmungsgemäßen Verwendung mehrfach verwendet werden kann, beispielsweise mindestens 5-mal, mindestens 10-mal oder sogar mindestens 50-mal, beispielsweise bis zu 500-mal oder mehr.

Der Einwegteil und der Mehrwegteil sind vorzugsweise reversibel miteinander verbindbar. Bei dieser Verbindung kann insbesondere das Auswerte- und Ansteuerteil oder ein Teil desselben gebildet werden, als eine Einheit, welche im Folgenden auch als Patch bezeichnet wird. Das Auswerte- und Ansteuerteil, vorzugsweise mit dem Einwegteil und dem Mehrwegteil, kann beispielsweise auf einer Hautoberfläche des Benutzers befestigbar sein, wobei beispielsweise das medizinische Funktionselement und/oder ein Teil desselben auch in das Körpergewebe hineinragen können, beispielsweise mittels einer Insertion. Die Verbindung zwischen Einwegteil und Mehrwegteil ist vorzugsweise reversibel ausgestaltet. Die Verbindung kann beispielsweise kraftschlüssig und/oder formschlüssig erfolgen. Zu diesem Zweck können beispielsweise der Einwegteil und/oder der Mehrwegteil jeweils ein oder mehrere Verbindungselemente umfassen. Beispielsweise kann eine Steckverbindung vorgesehen sein, mittels derer der Einwegteil und der Mehrwegteil zusammensteckbar sind, um die Einheit zu bilden. Auch eine Einheit, welche mehrere Einwegteile und/oder mehrere Mehrwegteile umfasst, ist realisierbar. Die Einheit aus Einwegteil und Mehrwegteil im verbundenen Zustand kann insbesondere im Wesentlichen feuchtigkeitsdicht ausgestaltet sein, um beispielsweise einen Innenraum dieser Einheit gegenüber Feuchtigkeit abzuschirmen.

Das Auswerte- und Ansteuerteil, insbesondere der Mehrwegteil, weist mindestens eine Ansteuerkomponente zur Steuerung der medizinischen Funktion auf. Beispielsweise kann das Auswerte- und Ansteuerteil mindestens ein Gehäuse aufweisen. Insbesondere kann der Mehrwegteil mindestens ein Mehrwegteilgehäuse aufweisen. Die mindestens eine Ansteuerkomponente kann in einem Inneren des mindestens einen Gehäuses, beispielsweise des Mehrwegteilgehäuses, angeordnet ist. Diese mindestens eine Ansteuerkomponente kann beispielsweise mindestens ein aktives elektronisches Bauelement umfassen, beispielsweise mindestens einen Operationsverstärker und/oder ähnliche elektronische Komponenten. Weiterhin kann die Ansteuerkomponente auch beispielsweise mindestens einen integrierten Schaltkreis umfassen. Unter einer Ansteuerkomponente ist dabei allgemein eine Komponente zu verstehen, welche eingerichtet ist, um die medizinische Funktion des medizinischen Funktionselements anzusteuern und/oder zu erfassen, beispielsweise indem das medizinische Funktionselement mit mindestens einem elektrischen Strom, mindestens einer elektrischen Spannung oder einem anderen Reiz beaufschlagt wird und/oder indem mindestens ein Signal, welches von dem medizinischen Funktionselement generiert wird, erfasst wird und beispielsweise umgewandelt und/oder gespeichert wird. Alternativ oder zusätzlich kann die mindestens eine Ansteuerkomponente auch mindestens eine mechanische, elektromechanische oder fluidische Komponente zur Unterstützung und/oder Steuerung der medizinischen Funktion umfassen, beispielsweise mindestens eine Pumpe, mindestens ein Rohrleitungssystem oder mindestens ein Ventil. Verschiedene andere Ausgestaltungen sind möglich und grundsätzlich aus dem Stand der Technik bekannt.

Das Auswerte- und Steuerteil weist mindestens ein Gehäuse und mindestens eine Batterieaufnahme auf. Beispielsweise kann das Einwegteil mindestens ein Einwegteilgehäuse aufweisen, und/oder das Mehrwegteil kann mindestens ein Mehrwegteilgehäuse aufweisen, wobei sich optional das Einwegteilgehäuse und das Mehrwegteilgehäuse beim Verbinden des Einwegteils und des Mehrwegteils zu dem Gehäuse des Auswerte- und Steuerteils oder eines Teils desselben ergänzen können, vorzugsweise flüssigkeitsdicht, luftdicht oder dampfdicht. Unter einem Gehäuse ist dabei allgemein im Rahmen der vorliegenden Erfindung ein mechanisches Bauteil zu verstehen, welches die Form eines durch das Gehäuse abgeschlossenen Teils, beispielsweise des Auswerte- und Steuerteils und/oder des Einwegteils, nach außen im Wesentlichen bestimmt und das Teil beispielsweise gegenüber mechanischen und/oder chemischen Einflüssen schützt und/oder abschirmt. Das Einwegteilgehäuse kann beispielsweise das mindestens eine Verbindungselement umfassen, mittels dessen der Einwegteil mit dem Mehrwegteil verbindbar ist, beispielsweise einen oder mehrere Steckverbinder und/oder Rastverbinder und/oder sonstige Verbindungselemente. Alternativ oder zusätzlich können das optionale Einwegteil und das optionale Mehrwegteil auch auf andere Weise als über ihre Gehäuse verbunden sein, beispielsweise über Steckverbinder oder andere Verbinder, welche nicht notwendigerweise Bestandteil des Gehäuses sind oder auf andere Weise. Das Einwegteilgehäuse kann insbesondere zusammen mit dem Mehrwegteil gemeinsam eine Einheit bilden, beispielsweise indem das Einwegteilgehäuse und das Mehrwegteilgehäuse komplementär ausgestaltet sind.

Die Batterieaufnahme kann als Bestandteil des Gehäuses, insbesondere des Einwegteilgehäuses, ausgestaltet sein oder lediglich mit dem Gehäuse, insbesondere dem Einwegteilgehäuse, verbunden sein. Unter einer Batterieaufnahme ist dabei ein Element zu verstehen, welches eingerichtet ist, um mindestens einen elektrischen Energiespeicher aufzunehmen. Die Batterieaufnahme weist bei der Vorrichtung mindestens einen derartigen elektrischen Energiespeicher auf. Beispielsweise kann dieser mindestens eine elektrische Energiespeicher mindestens eine Batterie umfassen. Auch andere Arten elektrischer Energiespeicher sind jedoch grundsätzlich einsetzbar, unabhängig von dem verwendeten Begriff der Batterieaufnahme. Die Batterieaufnahme kann beispielsweise mindestens einen Innenraum aufweisen, beispielsweise einen durch die Batterieaufnahme hermetisch nach außen abgeschirmten Innenraum, in welchem der elektrische Energiespeicher aufgenommen ist. Beispielsweise kann der Innenraum gegenüber einer Umgebung feuchtigkeitsdicht und/oder luftdicht und/oder wasserdampfdicht abgeschirmt sein.

Erfindungsgemäß wird vorgeschlagen, dass die Batterieaufnahme derart eingerichtet ist, dass diese durch den Benutzer irreversibel zur Entnahme des Energiespeichers geöffnet werden kann. Dieses Öffnen soll insbesondere ohne Verwendung von Werkzeugen erfolgen können, beispielsweise manuell. Unter einem irreversiblen Öffnen ist dabei ein Öffnen zu verstehen, bei welchem die Batterieaufnahme derart von dem Benutzer verformt wird, dass eine Rückführung in einen Ursprungszustand zumindest ohne Verwendung von Werkzeugen, Hilfsstoffen oder anderen Hilfsmitteln nicht möglich ist. Insbesondere kann ein hermetisch abgeschirmter Innenraum der Batterieaufnahme beim Öffnen derart irreversibel gegenüber der Umgebung geöffnet werden, dass ein erneutes Schließen des Innenraums, bei welchem die hermetische Abschirmung und/oder Versiegelung wiederhergestellt wird, ohne Hilfsmittel wie beispielsweise Klebstoffe, Werkzeuge oder ähnliches nicht möglich ist.

Die Batterieaufnahme kann insbesondere mindestens ein irreversibel zu öffnendes Verschlusselement umfassen, insbesondere ein durch den Benutzer zu öffnendes Verschlusselement, insbesondere mindestens eine Verschlussfolie, beispielsweise mindestens eine mit einem Batterieaufnahmegehäuse der Batterieaufnahme verklebte und/oder verschweißte Verschlussfolie. Diese Verschlussfolie kann beispielsweise den oben beschriebenen optionalen Innenraum der Batterieaufnahme hermetisch gegenüber einer Umgebung abdichten. Das Öffnen des Verschlusselements kann beispielsweise durch den Benutzer bewirkt werden. Das Öffnen kann beispielsweise mittels einer manuellen Handlung erfolgen. So kann der Benutzer beispielsweise die Verschlussfolie manuell abziehen. Alternativ oder zusätzlich kann das Öffnen des Verschlusselements auch derart eingerichtet sein, dass dieses automatisch oder halbautomatisch erfolgt, beispielsweise gekoppelt an mindestens eine andere Benutzerhandlung. Umfasst beispielsweise das Gehäuse, wie unten noch näher ausgeführt wird, ein Batterieaufnahmegehäuse, welches vom Gehäuse lösbar ist, so kann beispielsweise das Verschlusselement derart eingerichtet sein, dass dieses beim Lösen des Batterieaufnahmegehäuses vom Gehäuse automatisch geöffnet wird, beispielsweise indem eine Verschlussfolie automatisch abgezogen wird. Wiederum alternativ oder zusätzlich kann das Öffnen des Verschlusselements beispielsweise auch durch ein Trennen des Einwegteils und des Mehrwegteils erfolgen und/oder durch dieses Trennen ausgelöst werden. Wird beispielsweise ein Disposable vom Reusable getrennt, beispielsweise auf der Körperoberfläche oder in einem von der Körperoberfläche getrennten Zustand, so kann dieses Trennen insbesondere auch zu einem Öffnen des Verschlusselements führen, beispielsweise zu einem Öffnen der Verschlussfolie.

Die Batterieaufnahme kann insbesondere, wie oben beschrieben, mindestens ein Batterieaufnahmegehäuse aufweisen. Dieses Batterieaufnahmegehäuse und das Gehäuse, insbesondere das Einwegteilgehäuse, können miteinander verbunden sein, beispielsweise über eine kraftschlüssige und/oder formschlüssige Verbindung. Diese Verbindung zwischen dem Batterieaufnahmegehäuse und dem Gehäuse, insbesondere dem Einwegteilgehäuse, kann insbesondere derart ausgestaltet sein, dass diese durch den Benutzer lösbar ist, insbesondere irreversibel lösbar, beispielsweise unter Einwirkung üblicher manueller Kräfte des Benutzers. Dies kann insbesondere dergestalt erfolgen, dass das Batterieaufnahmegehäuse und/oder das Gehäuse, insbesondere das Einwegteilgehäuse, beim Lösen der Verbindung, welches insbesondere wiederum manuell erfolgen kann und ohne Zuhilfenahme von Werkzeugen, derart verformt werden, dass ein Wiederherstellen der Verbindung verhindert wird. Dies kann insbesondere derart erfolgen, dass beim Trennen des Batterieaufnahmegehäuses und/oder des Gehäuses, insbesondere des Einwegteilgehäuses, ein Knicken und/oder Zerreißen des Batterieaufnahmegehäuses und/oder des Gehäuses erfolgt, welches ein Wiederherstellen der Verbindung verhindert. Insbesondere kann das Gehäuse, vorzugsweise das Einwegteilgehäuse, mindestens eine Sollbruchstelle aufweisen, wobei das Gehäuse beim Lösen des Batterieaufnahmegehäuses von dem Gehäuse, insbesondere dem Einwegteilgehäuse, verformt wird, insbesondere irreversibel, so dass vorzugsweise eine Weiterbenutzung nicht möglich ist. Beispielsweise kann dies dergestalt erfolgen, dass die Sollbruchstelle mindestens eine Schwächung des Gehäuses, insbesondere des Einwegteilgehäuses, umfasst. Die Sollbruchstelle kann insbesondere derart ausgestaltet sein, dass, damit das Batterieaufnahmegehäuse von dem Gehäuse, insbesondere das Einwegteilgehäuse, gelöst werden kann, die Sollbruchstelle verformt, beispielsweise geknickt werden muss. Allgemein kann das Gehäuse, insbesondere das Einwegteilgehäuse, derart ausgestaltet werden, dass das Gehäuse bei der Verformung gebrochen und/oder geknickt wird.

Das Gehäuse, insbesondere das Einwegteilgehäuse, kann insbesondere mindestens eine Bodenplatte umfassen. Unter einer Bodenplatte ist dabei allgemein ein möglicherweise, jedoch nicht notwendigerweise plattenförmiges Element zu verstehen, welches eine Montageplattform für das Ansteuer- und Auswerteteil, insbesondere den Einwegteil, aufweist oder darstellt. Beispielsweise kann die Bodenplatte mindestens von der Hautoberfläche des Benutzers weg weisende ebene Fläche umfassen, auf welcher der optionale Einwegteil aufgebaut sein kann und auf welcher vorzugsweise auch der optionale Mehrwegteil montiert werden kann. Diese Bodenplatte kann insbesondere mindestens einen Aufnahmebereich zur Aufnahme des Batterieaufnahmegehäuses aufweisen. Insbesondere kann dieser Aufnahmebereich auf einer von einer Hautoberfläche des Benutzers weg weisenden Oberfläche der Bodenplatte angeordnet sein. Wie oben dargestellt, kann der Aufnahmebereich insbesondere derart ausgestaltet sein, dass das Batterieaufnahmegehäuse erst von dem Aufnahmebereich gelöst werden kann, wenn die Bodenplatte verformt worden ist, vorzugsweise irreversibel, beispielsweise entlang einer Sollbruchstelle, insbesondere einer Knicklinie. Beispielsweise kann das Batterieaufnahmegehäuse im Betriebszustand des Ansteuer- und Auswerteteils, insbesondere des Einwegteils, kraftschlüssig und/oder formschlüssig mit der Bodenplatte in dem Aufnahmebereich verbunden sein. Durch ein Knicken und/oder eine andere Art der irreversiblen Verformung der Bodenplatte kann diese formschlüssige und/oder kraftschlüssige Verbindung, welche beispielsweise eine Rastverbindung umfassen kann, lösbar sein, vorzugsweise irreversibel. Beispielsweise kann die Bodenplatte entlang mindestens einer Knicklinie knickbar sein, wobei durch das Knicken das Batterieaufnahmegehäuse von der Bodenplatte lösbar ist, beispielsweise indem eine kraftschlüssige und/oder formschlüssige Verbindung zwischen dem Batterieaufnahmegehäuse und der Bodenplatte freigegeben wird. Vorzugsweise tritt eine Verformung der Bodenplatte derart ein, dass diese nicht mehr weiterbenutzt werden kann. Beispielsweise kann dies wiederum durch Verwendung mindestens einer Sollbruchstelle erfolgen, beispielsweise der genannten Knicklinie.

Das Batterieaufnahmegehäuse kann insbesondere, wie oben ausgeführt, mindestens ein Verschlusselement umfassen. Dieses Verschlusselement kann beispielsweise mindestens eine Verschlussfolie umfassen. Das Verschlusselement kann insbesondere derart ausgestaltet sein, dass dieses erst nach der Lösung des Batterieaufnahmegehäuses von dem Einwegteilgehäuse für den Benutzer zur Entnahme des Energiespeichers aus der Batterieaufnahme geöffnet werden kann. Insbesondere kann die Batterieaufnahme im Betrieb der Vorrichtung, also bei mit dem Einwegteil verbundenem Batterieaufnahmegehäuse, für den Benutzer unzugänglich ausgestaltet sein. Die Ausgestaltung des Verschlusselements kann insbesondere derart sein, dass dieses erst nach der Lösung des Batterieaufnahmegehäuses von dem Gehäuse, insbesondere dem Einwegteilgehäuse, geöffnet werden kann, insbesondere durch den Benutzer. Dies kann beispielsweise dadurch erfolgen, dass das Verschlusselement auf einer dem Gehäuse, insbesondere dem Einwegteilgehäuse, zuweisenden Seite des Batterieaufnahmegehäuses angeordnet ist. Erst nach dem Lösen des Batterieaufnahmegehäuses von dem Gehäuse wird somit das Verschlusselement freigegeben. Die Lösung des Batterieaufnahmegehäuses von dem Gehäuse, insbesondere dem Einwegteilgehäuse, kann dabei vollständig oder auch teilweise erfolgen, wobei eine teilweise Lösung beispielsweise ein Aufklappen des Batterieaufnahmegehäuses, ein Aufschwenken oder ähnliches umfassen kann. Besonders bevorzugt ist es jedoch, wenn das Batterieaufnahmegehäuse vollständig von dem Gehäuse, insbesondere dem Einwegteilgehäuse, abnehmbar ist. Das Gehäuse, insbesondere das Einwegteilgehäuse, kann, wie oben beschrieben, beispielsweise mindestens eine Bodenplatte mit mindestens einem Aufnahmebereich zur Aufnahme des Batterieaufnahmegehäuses aufweisen. Bei in dem Aufnahmebereich aufgenommenem Batterieaufnahmegehäuse kann beispielsweise ein Formschluss und/oder Kraftschluss mit der Bodenplatte erfolgen, welcher beispielsweise durch ein Knicken und/oder Brechen entlang einer Sollbruchstelle, beispielsweise einer Knicklinie, lösbar sein kann. Erst nach diesem Brechen und/oder Knicken kann beispielsweise das Batterieaufnahmegehäuse derart freigegeben werden und derart von dem Gehäuse, insbesondere dem Einwegteilgehäuse, trennbar sein, dass das Verschlusselement durch einen Benutzer geöffnet werden kann, vorzugsweise wiederum rein manuell.

Die Vorrichtung kann insbesondere ganz oder teilweise mittels mindestens eines Befestigungselements, vorzugsweise mittels mindestens eines Pflasters und/oder einer anderen Art von selbstklebenden Oberfläche, auf eine Hautoberfläche des Benutzers fixierbar sein. Insbesondere kann, wie oben beschrieben, das Ansteuer- und Auswerteteil, insbesondere der Einwegteil, mindestens ein derartiges Befestigungselement, beispielsweise mindestens eine derartige selbstklebende Oberfläche und/oder mindestens ein derartiges Pflaster, umfassen. Beispielsweise kann das Ansteuer- und Auswerteteil, insbesondere der Einwegteil, wie oben beschrieben, mindestens eine Bodenplatte aufweisen, mit einer Auflagefläche, welche der Hautoberfläche des Benutzers zuweist und welche entweder selbstklebend ausgestaltet sein kann oder welche mittels mindestens eines klebenden Elements auf der Hautoberfläche fixierbar sein kann. Auf der Bodenplatte des Gehäuses des Ansteuer- und Auswerteteils, insbesondere auf der Bodenplatte des Einwegteilgehäuses des Einwegteils, kann dann beispielsweise direkt oder indirekt der optionale Mehrwegteil aufgesetzt und/oder angesetzt werden und mit dem optionalen Einwegteil verbunden werden. Dementsprechend kann die Vorrichtung beispielsweise vollständig oder teilweise als so genanntes Patch ausgestaltet sein, welches beispielsweise auf der Hautoberfläche aufklebbar ist. Ausgehend von dem Ansteuer- und Auswerteteil, insbesondere der Einheit aus Einwegteil und Mehrwegteil, können sich beispielsweise ein Sensor und/oder eine Kanüle vollständig oder teilweise in das Körpergewebe des Benutzers hinein erstrecken, also durch die Haut des Benutzers hindurch in das Körpergewebe.

Der optionale Einwegteil kann insbesondere derart ausgestaltet sein, dass dieses selbst keine elektronischen Bauelemente umfasst. Die Batterie bzw. der elektrische Energiespeicher sowie gegebenenfalls vorhandene Kontaktelemente und/oder Leiter sollen dabei nicht als elektrische Bauelemente zählen. Als elektrische Bauelemente sollen insbesondere passive elektrische Bauelemente wie beispielsweise elektrische Widerstände und/oder Dioden, und aktive elektrische Bauelemente wie beispielsweise Operationsverstärker und/oder Schaltkreise, insbesondere ASICs, zählen, welche vorzugsweise nicht in dem Einwegteil enthalten sind.

Wie oben dargestellt, kann das Funktionselement mindestens eine medizinische Funktion durchführen. Das Funktionselement kann insbesondere ausgewählt sein aus: einem Sensor zur Erfassung mindestens eines Körperzustands des Benutzers, insbesondere einem Sensor zur qualitativen und/oder quantitativen Erfassung mindestens eines Analyten in einer Körperflüssigkeit, insbesondere einem in ein Körpergewebe des Benutzers insertierbaren Sensor; einem Aktor zur Beeinflussung mindestens eines Körperzustands des Benutzers, insbesondere einem fluidischen Aktor zur Ausübung mindestens einer Medikationsfunktion, vorzugsweise einem Aktor mit mindestens einem in ein Körpergewebe des Benutzers insertierbaren Aktorteil, vorzugsweise mindestens einer Kanüle. Verschiedene andere Ausgestaltungen oder Kombinationen der genannten und/oder anderer Ausgestaltungen des Funktionselements sind möglich. So kann das Funktionselement insbesondere mindestens ein in das Körpergewebe insertierbares Teil umfassen, wie beispielsweise mindestens einen Sensor und/oder mindestens eine Kanüle.

Das Funktionselement kann insbesondere mit dem Ansteuer- und Auswerteteil, beispielsweise mit dem Einwegteil, verbindbar sein und/oder Bestandteil des Ansteuer- und Auswerteteils, beispielsweise des Einwegteils, sein. Die Verbindung kann reversibel oder auch irreversibel ausgestaltet sein.

Wie oben beschrieben, können der Mehrwegteil und der Einwegteil in verbundenem Zustand eine gemeinsame Einheit der Vorrichtung bilden. Dabei kann insbesondere ein gemeinsames Gehäuse entstehen, welches das Gehäuse des Ansteuer- und Auswerteteils oder eines Teils desselben bildet. Dieses gemeinsame Gehäuse kann insbesondere im Wesentlichen feuchtigkeitsdicht und/oder dampfdicht ausgestaltet sein, insbesondere um in dem Mehrwegteil und/oder in dem Einwegteil vorhandene Komponenten und/oder andere Komponenten der Ansteuer- und Auswertevorrichtung und/oder den elektrischen Energiespeicher gegenüber Feuchtigkeit zu schützen. Auch Verbindungselemente zwischen dem Mehrwegteil und dem Einwegteil können feuchtigkeitsdicht abgeschirmt sein, beispielsweise durch Verwendung entsprechender Dichtelemente wie beispielsweise O-Ringe. Auf diese Weise kann beispielsweise eine Steckverbindung zwischen dem Mehrwegteil und dem Einwegteil, welche neben einer rein mechanischen Steckfunktion auch eine elektrische Steckverbindung umfassen kann, feuchtigkeitsdicht abgeschirmt sein.

Neben der oben beschriebenen Vorrichtung in einer oder mehreren der beschriebenen Ausgestaltungen wird weiterhin ein Verfahren zur Entsorgung von Komponenten eines Ansteuer- und Auswerteteils, insbesondere eines Einwegteils, einer erfindungsgemäßen Vorrichtung in einer oder mehreren der oben beschriebenen Ausgestaltungen vorgeschlagen. Enthält das Ansteuer- und Auswerteteil mindestens ein Einwegteil und mindestens ein Mehrwegteil, so kann bei diesem Verfahren insbesondere eine Verbindung zwischen dem Einwegteil und dem Mehrwegteil getrennt werden. Weiterhin wird bei dem Verfahren eine Batterieaufnahme irreversibel zur Entnahme des Energiespeichers geöffnet, und der Energiespeicher wird der Batterieaufnahme entnommen. Der mindestens eine Energiespeicher kann anschließend getrennt von übrigen Komponenten des Ansteuer- und Auswerteteils, insbesondere des Einwegteils, entsorgt werden. Bei dem Verfahren kann insbesondere ein Batterieaufnahmegehäuse der Batterieaufnahme irreversibel von einem Gehäuse der Ansteuer- und Auswertevorrichtung, insbesondere einem Einwegteilgehäuse des Einwegteils, getrennt werden, insbesondere durch eine Verformung mindestens einer Sollbruchstelle. Anschließend kann die Batterieaufnahme irreversibel geöffnet werden, um den Energiespeicher zu entnehmen.

Die vorgeschlagene Vorrichtung und das vorgeschlagene Verfahren weisen gegenüber bekannten Vorrichtungen und Verfahren zahlreiche Vorteile auf. Insbesondere kann die Vorrichtung beispielsweise als Continuous Monitoring-Sensor für Glucose ausgestaltet sein, welche für einige Tage, Wochen oder sogar Monate am Körper des Benutzers getragen werden kann, währenddessen der Glucosesensor in das Körpergewebe des Benutzers implantiert sein kann. Der Sensor kann einen elektrischen Strom erzeugen, der in eine Spannung umgewandelt werden kann, die proportional zum Glucosemesswert ist. Für das Verstärken und Ausgeben des Messwerts kann insbesondere eine Funkverbindung vorgesehen sein, beispielsweise mittels mindestens eines in dem Ansteuer- und Auswerteteil, beispielsweise dem Mehrwegteil, enthaltenen Funkbausteins. Die hierfür benötigte elektrische Energie kann von dem in dem Ansteuer- und Auswerteteil, insbesondere dem Einwegteil, enthaltenen mindestens einen elektrischen Energiespeicher bereitgestellt werden, beispielsweise über eine elektrische Verbindung, insbesondere eine elektrische Steckverbindung, beispielsweise zwischen dem Einwegteil und dem Mehrwegteil. Für Funktionssicherheit und zur Erhaltung der Lebensqualität des Benutzers kann die komplette Vorrichtung während der Betriebszeit allen wirkenden Umwelteinflüssen, wie beispielsweise Feuchte, Temperaturschwankungen, mechanischen Einflüssen oder ähnlichen Einflüssen, widerstehen.

Durch die vorgeschlagene Entsorgungsmöglichkeit wird verhindert, dass der Benutzer den elektrischen Energiespeicher aufladen muss, wozu ein separates Ladegerät nötig wäre. Des Weiteren muss die typischerweise für 7 Tage oder mehr andauernde Langzeitmessung in der Regel nicht unterbrochen werden, beispielsweise indem ein Akku anfänglich nicht vollständig aufgeladen ist. Der Benutzer kann beispielsweise selbst den oben beschriebenen Entsorgungsvorgang vornehmen. Eine Rückübersendung der Vorrichtung bei leerer Batterie an den Hersteller, unter Austausch gegen eine neue Vorrichtung, ist nicht mehr erforderlich. Auf diese Weise wird auch vermieden, dass ein Hersteller oder ein separater Entsorgungsbetrieb kontaminierte Vorrichtungen erhält, welche dekontaminiert und dann mühsam wieder aufbereitet werden müssen.

Die Vorrichtung kann insbesondere als Bodymount ausgestaltet sein oder einen Bodymount umfassen, also als Bauelement, welches eingerichtet ist, um auf einer Haut des Benutzers fixiert zu werden. Der Bodymount kann insbesondere die Bodenplatte und die Batterieaufnahme umfassen. In dem Bodymount können sich beispielsweise ein oder mehrere Batterien befinden, welche beispielsweise in einem Einwegteil in einer Batterieaufnahme untergebracht sind, welche bei einer Entnahme der Batterien zerstört werden kann. Nach einer Messung, beispielsweise nach 7 Tagen Messzeit, kann der Benutzer beispielsweise den Originalverschluss öffnen und die Batterien entnehmen und separat entsorgen. Der Rest des Bodymounts kann im Hausmüll entsorgt werden.

Mittels der vorgeschlagenen Vorrichtung kann somit sichergestellt werden, dass der Benutzer stets frische Batterien zur Versorgung der Vorrichtung zur Verfügung hat, beispielsweise vor einer Messung. So kann beispielsweise verhindert werden, dass eine Messung mittels der Vorrichtung daran scheitert, dass die Batteriespannung zu gering ist, was bei längeren Benutzungsdauern, beispielsweise 7 Tagen Messzeit oder mehr, einen erheblichen Vorteil darstellt.

Die Vorrichtung, insbesondere das gemeinsame Gehäuse, welches insbesondere als Bodymount ausgestaltet ist, kann insbesondere eine hermetisch dichte Baugruppe bereitstellen. Der optionale Einwegteil kann ein Bodymount oder einen Teil desselben bilden, welches selbst separat geprüft werden kann, beispielsweise unabhängig von dem Mehrwegteil. Eine Rücksendung kontaminierter Baugruppen zum Hersteller ist in der Regel nicht erforderlich. Auch ein separates Ladegerät für die Vorrichtung bzw. den in dieser Vorrichtung enthaltenen elektrischen Energiespeicher ist nicht erforderlich. Der Benutzer selbst muss nicht in das vorzugsweise hermetisch dicht abgeschirmte System eingreifen. Da der Benutzer selbst die Batterie nicht in die Batterieaufnahme einlegt, entfällt auch das Risiko einer Batterieverpolung. Indem der Benutzer die Batterieaufnahme bei der Batterieentnahme vorzugsweise vollständig und insbesondere sichtbar zerstört, kann der Mehrwegteil und insbesondere die Batterieaufnahme des Mehrwegteils vorzugsweise nie wieder verwendet werden. Auf diese Weise wird auch ein Infektionsrisiko gesenkt.

Insbesondere die Ausgestaltung des Ansteuer- und Auswerteteils, beispielsweise des Einwegteils, mit mindestens einer Sollbruchstelle, beispielsweise in einer Bodenplatte, insbesondere einer Bodenplatte des Bodymounts, ist besonders bevorzugt, da eine derartige Sollbruchstelle für den Benutzer leicht abzuknicken ist. Durch dieses Abknicken kann beispielsweise eine Batterieaufnahme, insbesondere ein Batteriefach, zugänglich werden, um Batterien und/oder andere Arten von Energiespeichern zu entsorgen. Zum anderen kann das Ansteuer- und Auswerteteil und insbesondere der Einwegteil desselben, beispielsweise das Bodymount, auf diese Weise geometrisch derart verändert werden, dass eine Weiterbenutzung des Bodymounts unmöglich gemacht wird. Somit kann insbesondere eine für den Benutzer unzugängliche, hermetisch verschlossene Batterieaufnahme vorgesehen sein, welche am Ende der Nutzungszeit einfach für den Benutzer aufbrechbar und/oder auf andere Weise irreversibel geöffnet werden kann.

### Kurze Beschreibung der Figuren

Weitere Einzelheiten und Merkmale der Erfindung ergeben sich aus der nachfolgenden Beschreibung bevorzugter Ausführungsbeispiele, welche in den Figuren schematisch dargestellt sind. Gleiche Bezugsziffern in den einzelnen Figuren bezeichnen dabei gleiche oder funktionsgleiche bzw. hinsichtlich ihrer Funktionen einander entsprechende Elemente. Die Erfindung ist nicht auf die Ausführungsbeispiele beschränkt.

Im Einzelnen zeigen:
- Figur 1: ein Ausführungsbeispiel einer erfindungsgemäßen Vorrichtung mit einem Einwegteil und einem Mehrwegteil; und
- Figuren 2A bis 2C: verschiedene Verfahrensschritte eines Verfahrens zur Entsorgung von Komponenten des Einwegteils der Vorrichtung gemäß Figur 1.

### Ausführungsbeispiele

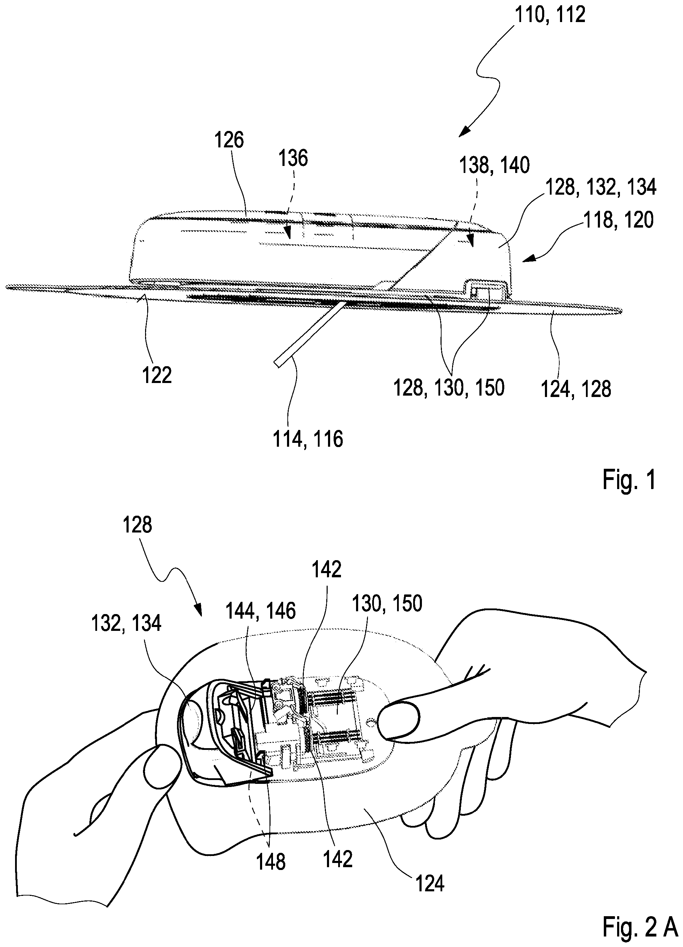

In Figur 1 ist in einer perspektivischen Darstellung ein Ausführungsbeispiel einer erfindungsgemäßen Vorrichtung 110 zur Durchführung mindestens einer medizinischen Funktion an einem Benutzer dargestellt. Die Vorrichtung 110 ist in dem dargestellten Ausführungsbeispiel als Sensorvorrichtung 112 für eine Langzeitüberwachung eines Analyten in einer Körperflüssigkeit, beispielsweise zur Glucoseüberwachung, ausgestaltet und weist ein medizinisches Funktionselement 114 in Form eines Sensors 116 auf, welcher beispielsweise als elektrochemischer Sensor ausgestaltet sein kann und welcher durch eine Hautoberfläche des Benutzers in ein Körpergewebe des Benutzers insertierbar ist. Der Sensor 116 ist mit einem auf einer Hautoberfläche des Benutzers montierbaren Gehäuse 118 eines Auswerte- und Steuerteils 120 verbunden, welches auch als Patch bezeichnet wird. Dieses Patch 120 weist eine der Hautoberfläche zuweisende selbstklebende Oberfläche 122 eines Pflasters 124 auf.

Der Patch 120 ist in dem dargestellten Ausführungsbeispiel optional mehrteilig ausgestaltet und umfasst in dem dargestellten Ausführungsbeispiel einen Mehrwegteil (Reusable) 126 und einen Einwegteil (Disposable) 128, zu welchem auch das Pflaster 124 gehört. Diese mehrteilige Ausgestaltung ist jedoch nicht zwingend erforderlich, so dass das dargestellte Beispiel auch derart modifiziert werden könnte, dass das Ansteuer- und Auswerteteil 120 einteilig ausgestaltet ist. Der Einwegteil 128 umfasst eine mit dem Pflaster 124 verbundene Bodenplatte 130, auf welche der Mehrwegteil 126 aufgesetzt ist. Weiterhin ist auf der Bodenplatte 130 ein Batterieaufnahmegehäuse 132 einer Batterieaufnahme 134 aufgebracht. Während der Mehrwegteil 126 eine in Figur 1 nicht näher dargestellte Ansteuer- und Auswerteelektronik 136 beinhaltet, ist in der Batterieaufnahme 134 mindestens ein elektrischer Energiespeicher 138 in Form beispielsweise mindestens einer Batterie 140, welche in Figur 1 ebenfalls nicht näher dargestellt ist, aufgenommen. Der Mehrwegteil 126 ist reversibel mit dem Einwegteil 128 verbunden, beispielsweise über einen oder mehrere Verbindungselemente 142, welche beispielsweise in der separaten Darstellung des Einwegteils 128 in Figur 2A erkennbar sind. Auf diese Weise kann beispielsweise eine hermetisch dichte Steckverbindung, welche lösbar ausgestaltet ist, zwischen dem Einwegteil 128 und dem Mehrwegteil 126 hergestellt werden, so dass das Patch 120 als gemeinsame Einheit entsteht.

Wie oben beschrieben, besteht eine Herausforderung von Vorrichtungen 110 wie der in Figur 1 dargestellten Vorrichtung insbesondere darin, dass zum einen der Benutzer nicht gezwungen sein soll, während der Benutzung der Vorrichtung 110 die Batterien 140 entweder auszutauschen oder aufzuladen, da die Batterieaufnahme 134 während der Benutzung gemeinsam mit der Bodenplatte 130 auf der Hautoberfläche des Patienten aufgeklebt wird. Zum zweiten soll trotz der geschlossenen Batterieaufnahme 134 gewährleistet sein, dass der Benutzer nach der Tragezeit die Batterien 140 separat entsorgen kann.

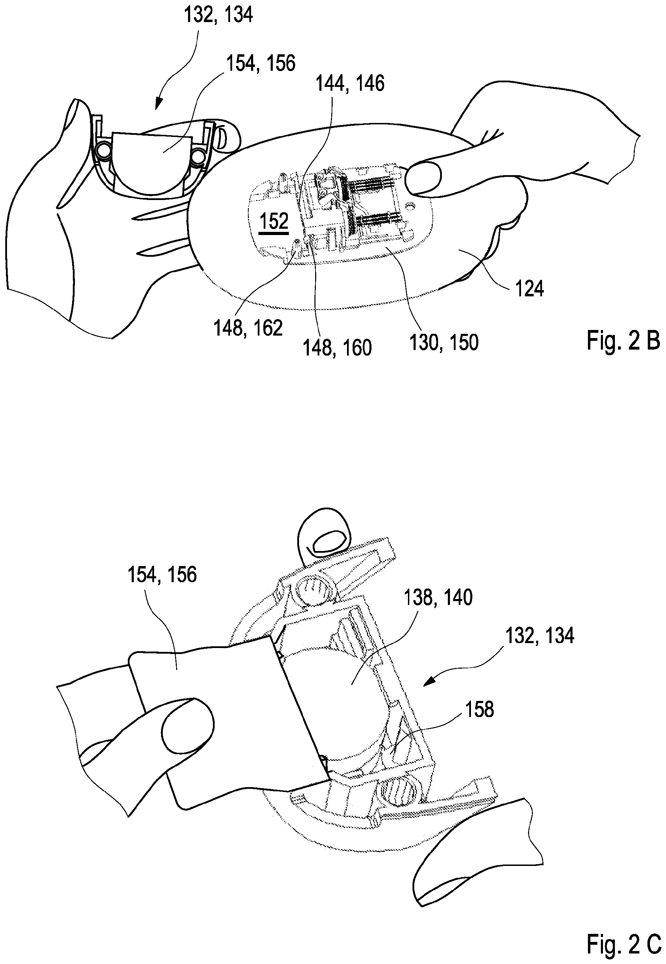

Dies wird durch eine spezielle Ausgestaltung des Einwegteils 128, welche in den Figuren 2A bis 2C erkennbar ist, gewährleistet. In Figur 2A ist dargestellt, dass die Bodenplatte 130 des Einwegteils 128 eine Sollbruchstelle 144 in Form einer Knicklinie 146 umfasst. Nach der Anwendung, beispielsweise einer Messdauer von 7 Tagen, kann der Benutzer den auch als Bodymount bezeichnete Einwegteil 128 samt Pflaster 124 von der Hautoberfläche abziehen und die Bodenplatte 130 entlang der Sollbruchstelle 124 so weit abknicken, bis sich die Batterieaufnahme 134, beispielsweise ein trennbares Batteriefach, von der Bodenplatte 130 löst. Bis dahin kann die Batterieaufnahme 134 mit ihrem Batterieaufnahmegehäuse 132 beispielsweise über ein oder mehrere Verbindungselemente 148 formschlüssig und/oder kraftschlüssig mit einem Einwegteilgehäuse 150, beispielsweise der Bodenplatte 130, verbunden sein, wobei diese Verbindung beim Abknicken irreversibel gelöst wird. Wie in Figur 2B dargestellt ist, kann dann das Batterieaufnahmegehäuse 132 der Batterieaufnahme 134 vollständig von der Bodenplatte 130 des Einwegteilgehäuses 150 gelöst werden.

Auf einer im Gebrauchszustand, also im nicht getrennten Zustand, einem Aufnahmebereich 152 der Bodenplatte 130 zuweisenden Seite der Batterieaufnahme 134 kann das Batterieaufnahmegehäuse 132 mindestens ein Verschlusselement 154 aufweisen, welches vorzugsweise irreversibel geöffnet werden kann. Insbesondere kann es sich hierbei um eine mit dem Batterieaufnahmegehäuse 132 verklebte und/oder verschweißte Verschlussfolie 156 handeln, welche transparent oder auch intransparent ausgestaltet sein kann und welche beispielsweise aus einem Kunststoffmaterial und/oder einem metallischen Material und/oder einem Laminatmaterial hergestellt sein kann. Durch Abziehen dieser Verschlussfolie 156, wie in Figur 2C dargestellt, kann die Batterie 140 aus der Batterieaufnahme 134 bzw. deren durch die Verschlussfolie 156 zuvor vorzugsweise hermetisch versiegeltem Innenraum 158 entnommen werden. Auf diese Weise ist eine separate Entsorgung der Batterien 140 möglich, unabhängig von der restlichen Entsorgung des Einwegteils 128.

In den Figuren 2B und 2C sind nochmals Details der Verbindungselemente 148 erkennbar. So ist erkennbar, dass diese Verbindungselemente einerseits eine Rastverbindung 160 umfassen können und andererseits beispielsweise auch, alternativ oder zusätzlich, eine Steckverbindung 162. Auch andere Ausgestaltungen sind jedoch grundsätzlich möglich. Durch das Knicken der Sollbruchstelle 144 entlang der Knicklinie 146 verliert die Bodenplatte 130 ihre starre Form, so dass selbst bei einer Wiederherstellung der Steckverbindung 162 die Rastverbindung 160 nicht wieder einrasten würde, so dass das Trennen der Batterieaufnahme 134 von der Bodenplatte 130 irreversibel erfolgt.

### Bezugszeichenliste

- 110: Vorrichtung zur Durchführung mindestens einer medizinischen Funktion
- 112: Sensorvorrichtung
- 114: medizinisches Funktionselement
- 116: Sensor
- 118: Gehäuse
- 120: Auswerte- und Steuerteil (Patch)
- 122: selbstklebende Oberfläche
- 124: Pflaster
- 126: Mehrwegteil (Reusable)
- 128: Einwegteil (Disposable)
- 130: Bodenplatte
- 132: Batterieaufnahmegehäuse
- 134: Batterieaufnahme
- 136: Ansteuer- und Auswerteelektronik
- 138: elektrischer Energiespeicher
- 140: Batterie
- 142: Verbindungselemente
- 144: Sollbruchstelle
- 146: Knicklinie
- 148: Verbindungselement
- 150: Einwegteilgehäuse
- 152: Aufnahmebereich
- 154: Verschlusselement
- 156: Verschlussfolie
- 158: Innenraum
- 160: Rastverbindung
- 162: Steckverbindung

## Patentansprüche

1. Vorrichtung (110) zur Durchführung mindestens einer medizinischen Funktion an einem Benutzer, wobei die Vorrichtung (110) mindestens ein medizinisches Funktionselement (114) aufweist, welches eingerichtet ist, um die medizinische Funktion durchzuführen, wobei die medizinische Funktion ausgewählt ist aus einer diagnostischen, einer therapeutischen und einer chirurgischen Funktion, wobei die Vorrichtung (110) mindestens ein Auswerte- und Steuerteil (120) aufweist, wobei das Auswerte- und Steuerteil (120) mindestens eine Ansteuerkomponente (136) zur Steuerung der medizinischen Funktion aufweist, wobei das Auswerte- und Steuerteil (120) mindestens ein Gehäuse (118) und mindestens eine Batterieaufnahme (134) aufweist, wobei die Batterieaufnahme (134) mindestens einen elektrischen Energiespeicher (138) umfasst, insbesondere mindestens eine Batterie (140), wobei die Batterieaufnahme (134) eingerichtet ist, um durch den Benutzer irreversibel zur Entnahme des Energiespeichers (138) geöffnet zu werden, wobei die Batterieaufnahme (134) ein Batterieaufnahmegehäuse (132) aufweist, wobei das Batterieaufnahmegehäuse (132) und das Gehäuse (118) miteinander verbunden sind, wobei die Verbindung durch den Benutzer irreversibel lösbar ist, wobei das Gehäuse (118), insbesondere das Einwegteilgehäuse (150), mindestens eine Sollbruchstelle (144) aufweist, wobei das Gehäuse (118) beim Lösen des Batterieaufnahmegehäuses (132) von dem Gehäuse (118) verformt wird.

2. Vorrichtung (110) nach dem vorhergehenden Anspruch, wobei das Auswerte- und Steuerteil (120) mindestens einen Einwegteil (128) und mindestens einen Mehrwegteil (126) aufweist, wobei der Einwegteil (128) und der Mehrwegteil (126) reversibel miteinander verbindbar sind, wobei der Mehrwegteil (126) die mindestens eine Ansteuerkomponente (136) zur Steuerung der medizinischen Funktion aufweist, wobei der Einwegteil (128) mindestens ein Einwegteilgehäuse (150) und die mindestens eine Batterieaufnahme (134) aufweist.

3. Vorrichtung (110) nach einem der vorhergehenden Ansprüche, wobei die Batterieaufnahme (134) mindestens ein irreversibel zu öffnendes Verschlusselement (154), insbesondere ein durch den Benutzer zu öffnendes Verschlusselement (154), umfasst.

4. Vorrichtung (110) nach einem der vorhergehenden Ansprüche, wobei die Verbindung derart ausgestaltet ist, dass das Batterieaufnahmegehäuse (132) und/oder das Gehäuse (118) beim Lösen der Verbindung derart verformt werden, dass ein Wiederherstellen der Verbindung verhindert wird.

5. Vorrichtung (110) nach einem der vorhergehenden Ansprüche, wobei das Gehäuse (118) bei der Verformung gebrochen und/oder geknickt wird.

6. Vorrichtung (110) nach einem der vorhergehenden Ansprüche, wobei das Gehäuse (118), insbesondere das Einwegteilgehäuse (150), mindestens eine Bodenplatte (130) umfasst, wobei die Bodenplatte (130) mindestens einen Aufnahmebereich (152) zur Aufnahme des Batterieaufnahmegehäuses (132) aufweist.

7. Vorrichtung (110) nach dem vorhergehenden Anspruch, wobei die Bodenplatte (130) die Sollbruchstelle (144) in Form mindestens einer Knicklinie (146) umfasst, wobei die Bodenplatte (130) entlang der Knicklinie (146) der Sollbruchstelle (144) knickbar ist, wobei durch das Knicken das Batterieaufnahmegehäuse (132) von der Bodenplatte (130) lösbar ist.

8. Vorrichtung (110) nach einem der vorhergehenden Ansprüche, wobei die Verbindung zwischen dem Gehäuse (118), insbesondere dem Einwegteilgehäuse (150), und dem Batterieaufnahmegehäuse (132) ausgewählt ist aus einer kraftschlüssigen und einer formschlüssigen Verbindung.

9. Vorrichtung (110) nach einem der vorhergehenden Ansprüche, wobei das Batterieaufnahmegehäuse (132) ein Verschlusselement (154) umfasst, insbesondere mindestens eine Verschlussfolie (156), wobei das Verschlusselement (154) derart ausgestaltet ist, dass dieses erst nach der Lösung des Batterieaufnahmegehäuses (132) von dem Gehäuse (118), insbesondere dem Einwegteilgehäuse (150), für den Benutzer zur Entnahme des Energiespeichers (138) aus der Batterieaufnahme (134) geöffnet werden kann.

10. Vorrichtung (110) nach einem der vorhergehenden Ansprüche, wobei das Funktionselement (114) ausgewählt ist aus: einem Sensor (116) zur Erfassung mindestens eines Körperzustands des Benutzers, insbesondere einem in ein Körpergewebe des Benutzers insertierbaren Sensor (116), vorzugsweise einem Sensor (116) zur Erfassung mindestens eines Analyten in einer Körperflüssigkeit des Benutzers; einem Aktor zur Beeinflussung mindestens eines Körperzustands des Benutzers, insbesondere einem fluidischen Aktor zur Ausübung mindestens einer Medikationsfunktion, vorzugsweise einem Aktor mit mindestens einem in ein Körpergewebe des Benutzers insertierbaren Aktorteil, vorzugsweise mindestens einer Kanüle.

11. Vorrichtung (110) nach einem der Ansprüche 2 - 10, wobei der Mehrwegteil (126) und der Einwegteil (128) in verbundenem Zustand ein gemeinsames Gehäuse (118) der Vorrichtung (110) bilden, vorzugsweise ein im Wesentlichen feuchtigkeitsdichtes Gehäuse (118).

12. Verfahren zur Entsorgung von Komponenten eines Auswerte- und Steuerteils (120) einer Vorrichtung (110) nach einem der vorhergehenden Ansprüche, wobei die Batterieaufnahme (134) irreversibel zur Entnahme des Energiespeichers (138) durch den Benutzer geöffnet wird, wobei der Energiespeicher (138) der Batterieaufnahme (134) entnommen wird, wobei der Energiespeicher (138) getrennt von übrigen Komponenten des Auswerte- und Steuerteils (120) entsorgt wird, wobei das Batterieaufhahmegehäuse (132) der Batterieaufnahme (134) irreversibel von dem Gehäuse (118) des Ansteuer- und Auswerteteils (120) getrennt wird durch eine Verformung der mindestens einen Sollbruchstelle (144), wobei anschließend die Batterieaufnahme (134) irreversibel geöffnet wird, um den Energiespeicher (138) zu entnehmen.

13. Verfahren nach dem vorhergehenden Anspruch, wobei das Auswerte- und Steuerteil (120) mindestens einen Einwegteil (128) und mindestens einen Mehrwegteil (126) aufweist, wobei der Einwegteil (128) und der Mehrwegteil (126) reversibel miteinander verbindbar sind, wobei der Mehrwegteil (126) die mindestens eine Ansteuerkomponente (136) zur Steuerung der medizinischen Funktion aufweist, wobei der Einwegteil (128) mindestens ein Einwegteilgehäuse (150) und die mindestens eine Batterieaufnahme (134) aufweist, wobei bei dem Verfahren die Verbindung zwischen dem Einwegteil (128) und dem Mehrwegteil (126) getrennt wird, wobei der Energiespeicher (138) getrennt von übrigen Komponenten des Einwegteils (128) entsorgt wird.

## Claims

1. Device (110) for performing at least one medical function on a user, wherein the device (110) has at least one medical functional element (114) that is designed to perform the medical function, wherein the medical function is selected from a diagnostic, a therapeutic and a surgical function, wherein the device (110) has at least one evaluation and control part (120), wherein the evaluation and control part (120) has at least one actuation component (136) for controlling the medical function, wherein the evaluation and control part (120) has at least one casing (118) and at least one battery receptacle (134), wherein the battery receptacle (134) comprises at least one electrical energy reservoir (138), more particularly at least one battery (140), wherein the battery receptacle (134) is designed to be opened irreversibly by the user for removing the energy reservoir (138), wherein the battery receptacle (134) has a battery-receptacle casing (132), wherein the battery-receptacle casing (132) and the casing (118) are interconnected, wherein the connection can be irreversibly detached by the user, wherein the casing (118), more particularly the disposable casing (150), has at least one predetermined breaking point (144), wherein the casing (118) is deformed when the battery-receptacle casing (132) is detached from the casing (118).

2. Device (110) according to the preceding claim, wherein the evaluation and control part (120) has at least one disposable (128) and at least one reusable (126), wherein the disposable (128) and the reusable (126) are reversibly interconnectable, wherein the reusable (126) has the at least one actuation component (136) for controlling the medical function, wherein the disposable (128) has at least one disposable casing (150) and the at least one battery receptacle (134).

3. Device (110) according to any one of the preceding claims, wherein the battery receptacle (134) comprises at least one closure element (154), wherein the closure element is to be opened in an irreversible fashion, more particularly a closure element (154), wherein the closure element is to be opened by the user.

4. Device (110) according to any one of the preceding claims, wherein the connection is embodied such that the battery-receptacle casing (132) and/or the casing (118) are deformed when the connection is detached such that re-establishing the connection is prevented.

5. Device (110) according to any one of the preceding claims, wherein the casing (118) is broken and/or kinked during the deformation.

6. Device (110) according to any one of the preceding claims, wherein the casing (118), more particularly the disposable casing (150), comprises at least one base plate (130), wherein the base plate (130) has at least one receiving region (152) for receiving the battery-receptacle casing (132).

7. Device (110) according to the preceding claim, wherein the base plate (130) comprises the predetermined breaking point (144) in the form of at least one kink line (146), wherein the base plate (130) is kinkable along the kink line (146) of the predetermined breaking point (144), wherein the battery-receptacle casing (132) is detachable from the base plate (130) as a result of kinking.

8. Device (110) according to any one of the preceding claims, wherein the connection between the casing (118), more particularly the disposable casing (150), and the battery-receptacle casing (132) is selected from a force-fit and an interlocking connection.

9. Device (110) according to any one of the preceding claims, wherein the battery-receptacle casing (132) comprises a closure element (154), more particularly at least one sealing film (156), wherein the closure element (154) is embodied such that it can only be opened for the user for removing the energy reservoir (138) from the battery receptacle (134) once the battery-receptacle casing (132) has been detached from the casing (118), more particularly the disposable casing (150).

10. Device (110) according to any one of the preceding claims, wherein the functional element (114) is selected from: a sensor (116) for registering at least one body state of the user, more particularly a sensor (116) that is insertable into a body tissue of the user, preferably a sensor (116) for detecting at least one analyte in a bodily fluid of the user; an actuator for influencing at least one body state of the user, more particularly a fluidic actuator for carrying out at least one medication function, preferably an actuator with at least one actuator part that is insertable into at least one body tissue of the user, preferably at least one cannula.

11. Device (110) according to any one of Claims 2-10, wherein the reusable (126) and the disposable (128) form a common casing (118) of the device (110) in a connected state, preferably a substantially moisture-tight casing (118).

12. Method for disposing of components of an evaluation and control part (120) of a device (110) according to any one of the preceding claims, wherein the battery receptacle (134) is irreversibly opened to remove the energy reservoir (138) by the user, wherein the energy reservoir (138) is removed from the battery receptacle (134), wherein the energy reservoir (138) is disposed of separately from the remaining components of the evaluation and control part (120), wherein the battery-receptacle casing (132) of the battery receptacle (134) is irreversibly separated from the casing (118) of the actuation and evaluation part (120) by a deforming the at least one predetermined breaking point (144), wherein the battery receptacle (134) is subsequently opened irreversibly to remove the energy reservoir (138).

13. Method according to the preceding claim, wherein the evaluation and control part (120) has at least one disposable (128) and at least one reusable (126), wherein the disposable (128) and the reusable (126) are reversibly interconnectable, wherein the reusable (126) has the at least one actuation component (136) for controlling the medical function, wherein the disposable (128) has at least one disposable casing (150) and the at least one battery receptacle (134), wherein the connection between the disposable (128) and the reusable (126) is separated during the method, wherein the energy reservoir (138) is disposed of separately from the remaining components of the disposable (128).

## Revendications

1. Dispositif (110) pour accomplir au moins une fonction médicale sur un utilisateur, le dispositif (110) possédant au moins un élément fonctionnel médical (114) qui est conçu pour accomplir la fonction médicale, la fonction médicale étant choisie parmi une fonction de diagnostic, thérapeutique et chirurgicale, le dispositif (110) possédant au moins une partie d'interprétation et de commande (120), la partie d'interprétation et de commande (120) possédant au moins un composant de commande (136) destiné à commander la fonction médicale, la partie d'interprétation et de commande (120) possédant au moins un boîtier (118) et au moins un logement de batterie (134), le logement de batterie (134) comportant au moins un accumulateur d'énergie électrique (138), notamment au moins une batterie (140), le logement de batterie (134) étant conçu pour être ouvert de manière irréversible par l'utilisateur en vue de retirer l'accumulateur d'énergie (138), le logement de batterie (134) possédant un boîtier de logement de batterie (132), le boîtier de logement de batterie (132) et le boîtier (118) étant assemblés l'un à l'autre, l'assemblage étant détechable défait de manière irréversible par l'utilisateur, le boîtier (118), notamment le boîtier partiel à usage unique (150), possédant au moins un point de rupture voulu (144), le boîtier (118) étant déformé lorsque le boîtier de logement de batterie (132) est détaché du boîtier (118).

2. Dispositif (110) selon la revendication précédente, la partie d'interprétation et de commande (120) possédant au moins une partie à usage unique (128) et au moins une partie réutilisable (126), la partie à usage unique (128) et la partie réutilisable (126) étant assemblables l'une à l'autre de manière réversible, la partie réutilisable (126) possédant l'au moins un composant de commande (136) destiné à commander la fonction médicale, la partie à usage unique (128) possédant au moins un boîtier partiel à usage unique (150) et l'au moins un logement de batterie (134).

3. Dispositif (110) selon l'une quelconque des revendications précédentes, le logement de batterie (134) comportant au moins un élément de fermeture (154) à ouvrir de manière irréversible, notamment un élément de fermeture (154) à ouvrir par l'utilisateur.

4. Dispositif (110) selon l'une quelconque des revendications précédentes, l'assemblage étant configuré de telle sorte que le boîtier de logement de batterie (132) et/ou le boîtier (118), lorsque l'assemblage est défait, sont déformés de telle sorte qu'un réassemblage de l'assemblage est empêché.

5. Dispositif (110) selon l'une quelconque des revendications précédentes, le boîtier (118) étant cassé et/ou plié lors de la déformation.

6. Dispositif (110) selon l'une quelconque des revendications précédentes, le boîtier (118), notamment le boîtier partiel à usage unique (150), comportant au moins une plaque de fond (130), la plaque de fond (130) possédant au moins une zone d'accueil (152) destinée à accueillir le boîtier de logement de batterie (132).

7. Dispositif (110) selon la revendication précédente, la plaque de fond (130) comportant le point de rupture voulu (144) sous la forme d'au moins une ligne de pliage (146), la plaque de fond (130) étant pliable le long de la ligne de pliage (146) du point de rupture voulu (144), le boîtier de logement de batterie (132) étant détachable de la plaque de fond (130) par le pliage.

8. Dispositif (110) selon l'une quelconque des revendications précédentes, l'assemblage entre le boîtier (118), notamment le boîtier partiel à usage unique (150), et le boîtier de logement de batterie (132) étant choisi parmi un assemblage par force ou un assemblage par complémentarité de formes.

9. Dispositif (110) selon l'une quelconque des revendications précédentes, le boîtier de logement de batterie (132) comportant un élément de fermeture (154), notamment au moins un film de fermeture (156), l'élément de fermeture (154) étant configuré de telle sorte que celui-ci peut seulement être ouvert après que le boîtier de logement de batterie (132) a été détaché du boîtier (118), notamment du boîtier partiel à usage unique (150), pour que l'utilisateur puisse retirer l'accumulateur d'énergie (138) hors du logement de batterie (134).

10. Dispositif (110) selon l'une quelconque des revendications précédentes, l'élément fonctionnel (114) étant choisi parmi : un capteur (116) destiné à détecter au moins un état corporel de l'utilisateur, notamment un capteur (116) étant insertable dans un tissu corporel de l'utilisateur, de préférence un capteur (116) destiné à détecter au moins une substance à analyser dans un fluide corporel de l'utilisateur ; un actionneur destiné à influencer au moins un état corporel de l'utilisateur, notamment un actionneur fluidique destiné à exercer au moins une fonction d'administration de médicament, de préférence un actionneur comprenant au moins une partie d'actionneur étant insertable dans un tissu corporel de l'utilisateur, de préférence au moins une canule.

11. Dispositif (110) selon l'une quelconque des revendications 2 à 10, la partie réutilisable (126) et la partie à usage unique (128) à l'état assemblé formant un boîtier (118) commun du dispositif (110), de préférence un boîtier (118) sensiblement étanche à l'humidité.

12. Procédé de mise au rebut de composants d'une partie d'interprétation et de commande (120) d'un dispositif (110) selon l'une quelconque des revendications précédentes, le logement de batterie (134) étant ouvert de manière irréversible par l'utilisateur en vue de retirer l'accumulateur d'énergie (138), l'accumulateur d'énergie (138) étant retiré du logement de batterie (134), l'accumulateur d'énergie (138) étant mis au rebut séparément des autres composants de la partie d'interprétation et de commande (120), le boîtier de logement de batterie (132) du logement de batterie (134) étant séparé de manière irréversible du boîtier (118) de la partie d'interprétation et de commande (120) par une déformation de l'au moins un point de rupture voulu (144), le logement de batterie (134) étant ensuite ouvert de manière irréversible afin de retirer l'accumulateur d'énergie (138).

13. Procédé selon la revendication précédente, la partie d'interprétation et de commande (120) possédant au moins une partie à usage unique (128) et au moins une partie réutilisable (126), la partie à usage unique (128) et la partie réutilisable (126) étant assemblables l'une à l'autre de manière réversible, la partie réutilisable (126) possédant l'au moins un composant de commande (136) destiné à commander la fonction médicale, la partie à usage unique (128) possédant au moins un boîtier partiel à usage unique (150) et l'au moins un logement de batterie (134), l'assemblage entre la partie à usage unique (128) et la partie réutilisable (126) étant défait lors du procédé, l'accumulateur d'énergie (138) étant mis au rebut séparément des autres composants de la partie à usage unique (128).
